# EUROPEAN PATENT APPLICATION

(11) **EP 4 442 821 A1**
(43) Date of publication of application: **09.10.2024**
(21) Application number: 23461550.8
(22) Date of filing: 05.04.2023
(51) Int. Cl.: C12N 11/04, A61K 35/747, C10B 53/00

(54) **METHOD OF IMMOBILIZING LACTOBACILLUS ACIDOPHILUS BACTERIA ON BIOCARBON BED AND USE OF BED WITH IMMOBILIZED LACTOBACILLUS ACIDOPHILUS BACTERIA**

(71) Applicant: Uniwersytet Zielonogórski, 65-417 Zielona Góra (PL)
(72) Inventor: Kliks, Jaroslaw, 66-120 Kargowa (PL); Korycka-Korwek, Justyna, 65-128 Zielona Gora (PL); Mrowczynska, Maria, 65-273 Zielona Gora (PL)
(74) Representative: JD&P Patent Attorneys Joanna Dargiewicz & Partners

(57) **Abstract**

The subject matter of the present invention is a method of immobilizing *Lactobacillus acidophilus* bacteria on a biocarbon bed, characterized in that it includes steps in which biocarbon is produced; *Lactobacillus acidophilus* cells are brought to a logarithmic growth phase; biocarbon is mixed with *Lactobacillus acidophilus* cells. The subject matter of the invention is also the biocarbon with immobilized *Lactobacillus acidophilus* bacteria obtained by this method, for use as a dietary supplement containing active microorganism forms, preferably to be administered in the form of capsules.

## Description

The present invention relates to a method of immobilizing *Lactobacillus acidophilus* bacteria on a biocarbon bed and a use of the bed with immobilized *Lactobacillus acidophilus* bacteria.

Biocarbon is a substance obtained by a process of pyrolysis of plants or other organic matter. It is characterized by high porosity and sorption capacity. In industrial applications, it is used to absorb odors, volatile substances, heavy metals and to decolorize liquid products.

The object of the present invention was to develop a method of obtaining biocarbon and immobilization inside its pores and on its surface the *Lactobacillus acidophilus* bacteria, which can thus be delivered to the small intestine.

### Prior art

The Czech patent CZ305666B6 discloses a method for producing a preparation based on biocarbon for promoting plant growth, which is based on the use of biocarbon in combination with cultured microorganisms. The phytomass intended for the preparation of biocarbon is heated in an induction heating reactor and the obtained biocarbon is then enriched with the addition of microorganisms and/or mycorrhizal fungi grown on biomass and/or fertilizers and/or hydrocolloids. The preparation obtained is used especially in agricultural and forestry practice to support the growth of higher plants.

Mexican patent application MX2012010137A describes a method for treating a biomass material, in particular a biomass material of plant origin, to pyrolyse it, comprising subjecting the biomass material to radiofrequency electromagnetic radiation, e.g. microwave radiation, while the material is mixed, in appropriate conditions to obtain the desired degree of pyrolysis. Pyrolysis temperature ranges from about 70°•C to 175°C (torrefaction). Coal, a solid product obtained in the process, used as feedstock for a coal-fired power plant. The application also relates to a compressed char product which is obtained by further treating the solid char product by mixing with a binder such as a binder, e.g. starch, and compacting the mixture to form a compressed product.

Taiwanese patent application TW202215979A discloses a method for producing biocarbon, which includes burning plant biomass materials and carbonizing plant biomass materials; grinding charred materials from plant biomass and obtaining biocarbon with a particle size in the range of 10 micrometers to 1000 micrometers. The application also relates to a feed additive containing biocarbon and microorganisms which are selected from Bacillus, photosynthetic bacteria, lactobacilli, yeast and Vibrio. Also disclosed is a method of feeding the feed, the use of a feed additive to produce an aquaculture feed, and a method for purifying water.

In turn, Chinese patent application CN113234714A relates to a microbial inoculum that promotes plant growth, including functional flora that promotes plant growth as the main ingredient, corn straw biocarbon as an adsorbent, polyvinyl alcohol as a carrier, sodium alginate and Ca(NO₃)₂ as a cross-linking agent for fixing.

Another Chinese patent application CN111670750A relates to the microbial inoculant of the Rhizophagus intraradices-biocarbon complex and the method of its production and use. The complex microbial inoculant comprises Rhizophagus intraradices and biocarbon in a mass ratio (4-15:1), where Rhizophagus intraradices is obtained by isolation from the rhizospheres of Solanaceae plants and biocarbon is rice straw biocarbon. The microbial inoculant increases the emergence rate of pepper seedlings, plant height, stem thickness, aboveground biomass, root biomass and fresh fruit weight per plant.

The next Chinese patent application CN106365139A discloses biocarbon prepared from a harvest of plants refined in a single-family backyard. The production process includes steps in which the Fe2+-enriched plant biomass is dried and ground; broken plant particles are placed in a carbon dioxide atmosphere, the temperature is raised to 800-900°C from room temperature in 150-200 minutes and held for 100-200 minutes, after which the temperature is lowered to room temperature in 150-200 minutes, and biomass is carbonized; biocarbon formed after carbonization is subjected to microwave radiation with a microwave radiation power of 300 W-700 W, and then soaked, washed and activated with zinc chloride; the biocarbon obtained after activation is dried and sodium borohydride solution is added dropwise in an ice-water bath to reduce the ferrous ions to zero valence iron; followed by washing with water until neutral pH and drying to obtain activated biocarbon.

Another Chinese patent application CN113980695A relates to a method for treating plant biomass enriched with heavy metals. Plants growing in soil contaminated with heavy metals are subjected to pyrolysis, biomass is subjected to pyrolysis to biocarbon at a temperature of 400-800°C, followed by extraction of biocarbon using a solution of nitric acid with a pH of 1-3.

Another Chinese patent application CN114456811A relates to a carbon-based soil remediation agent and a method for its production. Farmland carbon based soil remediation agent contains 10%-25% modified biocarbon and 75%-90% beneficial bacteria by weight, the beneficial bacteria include *Lactobacillus plantarum, Lactobacillus acidophilus, Saccharomyces cerevisiae, Bacillus subtilis, Bacillus licheniformis and Bacillus megatherium.*

### Summary of the invention

Thus, the subject matter of the present invention is a method of immobilizing *Lactobacillus acidophilus* bacteria on a biocarbon bed, characterized in that it includes steps in which:
a) biocarbon is produced;
b) *Lactobacillus acidophilus* cells are brought to a logarithmic growth phase;
c) the biocarbon is mixed with *Lactobacillus acidophilus* cells, wherein
   in step a)
      - pomace, preferably oligosaccharide-rich deoiled flax or coconut pomace, is placed in a microwave dryer on a conveyor belt, with a belt speed of 0.15-0.25 m/minute, microwave frequency in the range of 2.40 to 2.45 GHz, for a period of 1 hour, at a power of 20 kW;
      - the obtained matter is subjected to a vacuum process in a chamber at 150°C, at a pressure of 10-400 Pa for 10 hours with the outlet valve open, followed by a thermal process according to the temperature program: 1 h - 300°C, 8 h - 650-700°C with the outlet valve closed;
      - after this time, the chamber is cooled to 200°C and the pressure is equalized to ambient pressure;
      - the obtained biocarbon is washed to remove mineral deposits;
   in step b)
      - freeze-dried *Lactobacillus acidophilus* cells in the amount of 20 g are placed in the fermentation tank in 1 L of medium solution containing 40 g/L of lactose, 50 mg/L of magnesium ions Mg²⁺, 100 mg/L of sodium ions Na⁺, 90 mg/L of calcium ions Ca²⁺, and 0.5 mg/L of chromium ions Cr²⁺;
      - the mixture of cells in the medium is heated to 25°C with continuous stirring at 150 RPM and the fermentation process is carried out for 8 hours;
      - the medium is successively added to the fermentation tank in the amount of 150 mL per hour for further 3 hours;
   in step c)
      - 200 g of the biocarbon obtained in step a) is added to the fermentation tank from step b) and the fermentation is continued, adding 500 mL of medium every hour for further 3 hours;
      - another 200 g of biocarbon is added to the fermentation tank, the whole is mixed and left for 1 hour, after which the biocarbon containing the absorbed biomass is centrifuged.

Preferably, the method additionally includes step d) of encapsulating the biocarbon containing the absorbed biomass.

Preferably, in step d):
- 400 g of biocarbon containing the absorbed biomass is added to 2 L of a solution composed of: 40 g/L of lactose, 25 g/L of sodium alginate, 5 g/L of sodium carbonate, 0.2 g/L of zinc sulfate (VI) and the whole is mixed for 20 minutes at 150 RPM;
- then the biomass-containing biocarbon suspension is dispensed by microdroplets with a droplet size of 20 to 45 µL into a high-speed mixer containing a 2 wt% calcium chloride (CaCl₂) solution;
- the obtained encapsulated particles are centrifuged, washed with distilled water and separated.

Preferably, the method additionally includes step e) of freezing and freeze-drying the encapsulated biocarbon particles containing the absorbed biomass.

Preferably, in step e), the encapsulated particles obtained in step d) are subjected to shock freezing at -75°C, followed by a freeze-drying process according to the following scheme:
- 24 h, temperature 20°C, pressure 200 Pa,
- 48 h, temperature 32°C, pressure 20 Pa.

The subject matter of the present invention is also a biocarbon with immobilized *Lactobacillus acidophilus* bacteria obtained by the above-defined method, for use as a dietary supplement containing active microorganism forms, preferably to be administered in the form of capsules.

### Detailed description of the invention

Biocarbon is characterized by varying porosity and unpredictable binding capacity of absorbed substances. In order to be able to control and standardize the sorption capacity of biocarbon, a method was developed to fix the absorbed bacterial biomass inside the pores of biocarbon. Thus produced substance can have use in pharmacy as a bioactive carrier of the so-called probiotic flora, and in the food industry as a carrier of biologically active microorganisms. The produced bed can support the transport of bacterial flora into the intestines. Conditions in the gastrointestinal tract, especially the low pH in the stomach, make it difficult for positive intestinal flora to get to their final habitat. With the help of a bed with immobilized *Lactobacillus acidophilus* bacteria, it is possible for live bacteria to enter the small intestine.

### Example 1

### Biocarbon production

In order to obtain a biocarbon bed, the pomace, in this embodiment an oligosaccharide-rich deoiled flax or coconut pomace, was placed in a microwave dryer on a conveyor belt. The belt speed was 0.25 m/minute, the microwave frequency in the range of 2.40 to 2.45 GHz, the process duration was 1 hour, and the power was 20 kW. As a result of the above process, largely carbonized matter was obtained, which was subjected to a vacuum process in a steel chamber, at 150°C, pressure 10 Pa, for 10 hours. This process has a positive effect on the removal of volatile organic compounds. The outlet valve was then closed and the thermal process was carried out according to the temperature program: 1 h - 300°C, 8h - 700°C. After this time, the chamber was cooled to 200°C and the pressure control valve was opened to equalize the pressure with the environment. The obtained biocarbon was washed to remove mineral deposits.

### Example 2

### Immobilization of bacterial biomass on biocarbon

Preferably the microorganism is brought to the so-called logarithmic growth phase before the actual immobilization process. This is a state in which there is a dynamic increase in the number of the microorganism cells. For *Lactobacillus acidophilus* bacilli, the logarithmic (dynamic) growth phase was achieved as follows:
Freeze-dried *Lactobacillus acidophilus* cells in the amount of 20 g were transferred to 1 dm³ of medium solution containing 40 g/L of lactose, 50 mg/L of magnesium ions Mg²⁺, 100 mg/L of sodium ions Na⁺, 90 mg/L of calcium ions Ca²⁺, and 0.5 mg/L of chromium ions Cr²⁺. The solution was heated to 25°C with constant stirring (150 RPM). The fermentation was carried out for 8 hours. After this time, media were successively added to the fermentation tank in the amount of 150 mL of medium per hour. In this way, the process was carried out using the fed-batch method for further 3 hours. After this time, 200 g of biocarbon was added to the total volume of 1.45 L of medium obtained, and fermentation was continued by adding 500 mL of medium every hour for further 3 hours. After 3 hours, 200 g of biocarbon was added, the total was mixed and left for 1 hour, and then the biocarbon was centrifuged.

The obtained 400 g of biocarbon containing the absorbed biomass was transferred to a beaker containing 2 L of a solution composed of: 40 g/L of lactose, 25 g/L of sodium alginate, 5 g/L of sodium carbonate, 0.2 g/L of zinc sulfate (VI). The solution was stirred for 20 minutes at 150 RPM.

The biomass-containing biocarbon suspension was then dispensed by microdroplets (droplet size 20-45 µL) into a high-speed mixer containing a 2 wt% calcium chloride (CaCl₂) solution.

Interactions between the alginate and calcium ions result in the formation of coats of the sparingly soluble form of calcium alginate. Inside the coats there is a solution containing particles of biocarbon with biomass absorbed on its surface. The encapsulated particles thus produced were centrifuged and washed three times in distilled water, and then separated on sieves.

The biomass obtained was subjected to shock freezing at -75°C, followed by freeze-drying process according to the following scheme:
- 24 h, temperature 20°C, pressure 200 Pa,
- 48 h, temperature 32°C, pressure 20 Pa.

The obtained powder can be administered in the form of capsules as a supplement containing active microorganism forms.

### Example 3

### Biological tests of the obtained biocarbon powders

The results of microbiological cultures for the obtained biocarbon powders with immobilized lactobacilli are shown in Table 1. The results of the cultures indicate a high biomass content of *Lactobacillus acidophilus* cells in the analyzed material. Thus, the tested material could be an ingredient of functional food or an ingredient of dietary supplement, in which it acts as a carrier of probiotic *Lactobacillus acidophilus* bacteria.

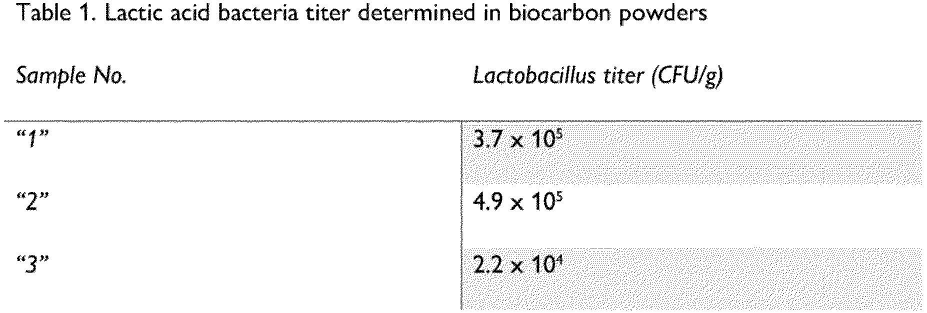

## Claims

1. A method of immobilizing *Lactobacillus acidophilus* bacteria on a biocarbon bed, **characterized in that** it includes steps in which:
a) biocarbon is produced;
b) *Lactobacillus acidophilus* cells are brought to the logarithmic growth phase;
c) the biocarbon is mixed with *Lactobacillus acidophilus* cells, wherein
in step a)
- pomace, preferably oligosaccharide-rich deoiled flax or coconut pomace, is placed in a microwave dryer on a conveyor belt, with a belt speed of 0.15-0.25 m/minute, microwave frequency in the range of 2.40 to 2.45 GHz, for a period of 1 hour, at a power of 20 kW;
- the obtained matter is subjected to a vacuum process in a chamber at 150°C, at a pressure of 10-400 Pa for 10 hours with the outlet valve open, followed by a thermal process according to the temperature program: 1 h - 300°C, 8 h - 650-700°C with the outlet valve closed;
- after this time, the chamber is cooled to 200°C and the pressure is equalized to ambient pressure;
- the obtained biocarbon is washed to remove mineral deposits;
in step b)
- freeze-dried *Lactobacillus acidophilus* cells in the amount of 20 g are placed in the fermentation tank in 1 L of medium solution containing 40 g/L of lactose, 50 mg/L of magnesium ions Mg²⁺, 100 mg/L of sodium ions Na⁺, 90 mg/L of calcium ions Ca²⁺, and 0.5 mg/L of chromium ions Cr²⁺;
- the mixture of cells in the medium is heated to 25°C with continuous stirring at 150 RPM and the fermentation process is carried out for 8 hours;
- the medium is successively added to the fermentation tank in the amount of 150 mL per hour for further 3 hours;
in step c)
- 200 g of the biocarbon obtained in step a) is added to the fermentation tank from step b) and the fermentation is continued, adding 500 mL of medium every hour for further 3 hours;
- another 200 g of biocarbon is added to the fermentation tank, the whole is mixed and left for 1 hour, after which the biocarbon containing the absorbed biomass is centrifuged.

2. The method according to claim 1, **characterized in that** it additionally includes step d) of encapsulating the biocarbon containing the absorbed biomass.

3. The method according to claim 2, **characterized in that** in step d):
- 400 g of biocarbon containing the absorbed biomass is added to 2 L of a solution composed of: 40 g/L of lactose, 25 g/L of sodium alginate, 5 g/L of sodium carbonate, 0.2 g/L of zinc sulfate (VI) and the whole is mixed for 20 minutes at 150 RPM;
- then the biomass-containing biocarbon suspension is dispensed by microdroplets with a droplet size of 20 to 45 µL into a high-speed mixer containing a 2 wt% calcium chloride (CaCl₂) solution;
- the obtained encapsulated particles are centrifuged, washed with distilled water and separated.

4. The method according to claim 2 or 3, **characterized in that** it additionally includes step e) of freezing and freeze-drying the encapsulated biocarbon particles containing the absorbed biomass.

5. The method according to claim 4, **characterized in that** in step e) the encapsulated particles obtained in step d) are subjected to shock freezing at -75°C, followed by a freeze-drying process according to the following scheme:
- 24 h, temperature 20°C, pressure 200 Pa,
- 48 h, temperature 32°C, pressure 20 Pa.

6. Biocarbon with immobilized *Lactobacillus acidophilus* bacteria obtained by the method defined in any of the claims 1 to 5, for use as a dietary supplement containing active microorganism forms.

7. The biocarbon according to claim 6 to be administered in the form of capsules.
